# EUROPEAN PATENT SPECIFICATION

(11) **EP 0 939 086 B1**
(45) Date of publication and mention of the grant of the patent: **10.03.2004**
(21) Application number: 98200620.7
(22) Date of filing: 27.02.1998
(51) Int. Cl.: C08B 37/08, A61L 31/00

(54) **Process for producing crosslinked hyaluronic acid**
Verfahren zur Herstellung von vernetzter Hyaluronsäure
Procédé pour préparer un acide hyaluronique réticulé

(43) Date of publication of application: 01.09.1999
(73) Proprietor: Stichting Hippomedics, 9541 BV Vlagtwedde (NL)
(72) Inventor: Van der Tuin, Everhardus, 9541 BG Vlagtwedde (NL); Besemer, Arie Cornelis, 3958 CC Amerongen (NL)
(74) Representative: van Westenbrugge, Andries

(56) References cited:
- GB-A- 2 151 244
- US-A- 4 582 865

## Description

The invention relates to a process for producing crosslinked hyaluronic acid by treating an aqueous solution of hyaluronic acid with divinyl sulphone.

Hyaluronic acid (HA) is a high molecular weight, straight-chain mucopolysaccharide consisting of repeating glucuronic acid - N-acetylglucosamine disaccharide units. It has a high water-absorbing capacity. HA is a component of e.g. skin, connective tissue and vitreous humour of the eye. HA is also present in the synovial fluid of joints, where it has a function as a lubricant and as a shock absorber.

A production deficiency of HA results in painful joints (osteoarthritis) in man and in lameness in horses. Apart from being a medical problem, lameness in horses is also an economical problem, especially for racing horses.

For several years now, lameness has been controlled by viscosupplementation, which involves injecting a gel of HA into the joint. The injected amount of HA is usually 16-40 mg in several millilitres of neutral water. A commercially available product is a synovial viscosupplementation HA gel/fluid of 20 mg referred to as Synvisc GF20. The HA used in this product can be obtained by crosslinking HA with divinyl sulphone (DVS) at high pH (0.2 M sodium hydroxide) and at 20°C, using a HA/DVS weight ratio between 15:1 and 1:5, as described in US Patent 4,582,865. Crosslinked HA which is suitable for intra-articular injection can also be obtained by crosslinking HA with an excess of bifunctional epoxides, such as epichlorohydrine, as disclosed in EP-A-0161887.

Although initially the use of the prior art HA products such as Synvisc in joint treatment of horses is satisfactory, the symptoms of joint failure return after a few months, often preceded by inflammatory responses.

It has been found now that a crosslinked HA can be obtained which is in the form of a viscous solution rather than a gel and which has an improved long-term performance in joint treatment, if the crosslinking of HA is carried out under specific conditions as defined in the appending claims.

An important feature of the process of the invention is the low ratio of crosslinking agent and HA. The weight ratio of HA (based on the sodium salt) to DVS should be between 20 and 200, preferably between 40 and 120. A weight ratio HA/DVS (WR_{HA/DVS}) of 20 corresponds to a molar percentage of DVS to HA (MP_{DVS/HA}) of 17% (on the basis of an anhydro[sodium glucuronate - N-acetylglucosamine] disaccharide) or 8.5% (on average anhydromonosaccharide basis). Thus, the molar percentage of crosslinking agent to HA (monosaccharide basis) should be between about 0.9 and 8.5, preferably between about 1.7 and 5. The solution to be crosslinked may contain other biopolymers (polysaccharides, proteins, glycoproteins etc.) in addition to HA, but preferably less than one third of the amount of HA, most preferably less than 5% thereof.

Another important feature is the pH of the crosslinking solution. It should be below 11, preferably above 7.5 and more preferably between 8 and 11. It was found that the use of a higher pH results in HA solutions which cause allergic and inflammatory reactions, possibly due to degradation of HA. Also it is preferred that the crosslinking is carried out at relatively low temperatures, such as below 15°C, especially between 0 and 12°C.

The crosslinked HA preferably has a molecular weight between 0.7 and 8 MDa, the average molecular weight preferably being between 1.2 and 5 MDa. It is further preferred that the crosslinked HA is present in a viscous solution and not in a gel. The viscosity of the solution is preferably between 15,000 and 40,000 cP, most preferably between 18,000 and 32,000 cP, as measured with a Brookfield viscosimeter.

The invention also pertains to the viscous solution thus obtained and to its use in viscosupplementation of joints, especially for horses, and in ophthalmic surgery, especially as a temporary filling of the ey socket during surgery.

### Example 1

To 100 ml of a solution of HA (Sigma, *Streptococcus zooepidemicus*, 0.8% w/w in water) at pH 8.5 and 5°C, 15 mg of DVS was added (WR_{HA/DVS}= = 53). The mixture was homogenised and allowed to stand at 4-6°C for one week. The solution was then adjusted to pH 7 using acetic acid and sterilised by filtration (0.45 µm).
The solution thus obtained was injected into the joints of racing horses submitted to high stress conditions. The animals treated with this solution were free of joint problems until about 6 months after injection, when the treatment was repeated. Animals treated with prior art HA compositions (Synvisc) needed repeat of injection after 2 or 3 months.

### Example 2

To 100 ml of a solution of HA (Shiseido, *Streptococcus zooepidemicus*, 0.8% w/w in water) at pH 8.5 and 5°C, 10 mg of DVS was added (WR_{HA/DVS} = 80). The solution was treated as described in example 1 and also performed better than the prior art material.

### Example 3

To 100 ml of a solution 1 g of containing HA (Sigma, *Streptococcus zooepidemicus*) and 20 mg of DVS (WR_{HA/DVS} = 50) was added 200 mg of finely powdered sodium carbonate under stirring at pH 7. After a clear solution had been obtained, stirring was discontinued. After the sodium carbonate had been added, the pH was 10. The mixture was allowed to stand for 3 days at 4-6°C. Then the solution was carefully neutralised with acetic acid (pH 7) and further treated as described in example 1. Apart from a slight swelling at about 10% of the horses, this product was equally satisfactory as the product of example 1. The viscosities and results of treatments of various crosslinked solutions is given in the table below:

**Table**

| degree of crosslinking (mol%) | viscosity (cP) | number animals tested | result (treatment) ¹) | result (inflammatory response)²) |
|---|---|---|---|---|
| 0 | 14,000 | 1000 | + | ++ |
| 2 | 18,000 | 30 | ++ | + |
| 3 | 22,000 | 30 | ++ | + |
| 4 | 25,000 | 30 | ++ | + |

| | | | | |
|---|---|---|---|---|
| ¹) + means: symptoms dissappear, horse can be trained within 1-2 days, remedication necessary after 3-5 months in case of intensive training; ++ means: symptoms disappear, horse can be trained within 2 days, remedication necessary after 6-24 months in case of intensive training. | | | | |
| ²) + means: less than 3% showing inflammations; disppeared within 24 hours; ++ means: less than 0.5% showing inflammations; disppeared within 24 hours. | | | | |

When this example was repeated using 0.2 M NaOH during crosslinking, vigorous inflammatory responses (swellings) occurred, which could only be treated with corticosteroids.

## Claims

1. A process for producing a crosslinked hyaluronic acid (HA) or a salt thereof, by crosslinking an aqueous solution of HA with divinyl sulphone (DVS), ***characterised* in that** the crosslinking with DVS is carried out at a pH between 8 and 11 using a DVS/HA molar ratio between 1 and 10% (a mole of HA being equivalent to an average anhydromonosaccharide unit).

2. A process according to claim 1, wherein a DVS/HA molar weight ratio between 2 and 5% is applied.

3. A process according to claim 1 or 2, wherein the crosslinked HA has a molecular weight of between 0.7 and 8 MDa.

4. A process according to any one of claims 1-3, wherein the crosslinking is carried out at a temperature between 0 and 12°C.

5. A process according to any one of claims 1-4, wherein the crosslinked hyaluronic acid is produced in the form of a viscous aqueous solution.

6. A process according to claim 5, wherein the solution has a viscosity of 15,000-40,000 cP.

7. A viscous aqueous solution of crosslinked hyaluronic acid or salt thereof, suitable for viscosupplementation of joints, said crosslinked hyaluronic acid having a molecular weight of between 0.7 and 8 MDa and said solution having a viscosity between 15,000 and 40,000 cP.

8. A viscous solution according to claim 7, having a viscosity between 18,000 and 32,000 cP.

9. Use of a viscous solution according to claim 7 or 8, or obtained according to any one of claims 1-6, for use in human or veterinary therapy or surgery.

## Patentansprüche

1. Verfahren zur Herstellung einer vernetzten Hyaluronsäure (HS) oder eines Salzes davon durch das Vernetzen einer wäßrigen Lösung von HS mit Divinylsulfon (DVS), **dadurch gekennzeichnet, daß** das Vernetzen mit DVS bei einem pH zwischen 8 und 11 unter Verwendung eines DVS/HS-Molverhältnisses zwischen 1 und 10% (wobei ein Mol HS einer durchschnittlichen Anhydromonosaccharideinheit äquivalent ist) durchgeführt wird.

2. Verfahren nach Anspruch 1, wobei ein DVS/HS-Molgewichtsverhältnis zwischen 2 und 5% angewendet wird.

3. Verfahren nach Anspruch 1 oder 2, wobei die vemetzte HS ein Molekulargewicht von zwischen 0,7 und 8 MDa aufweist.

4. Verfahren nach einem der Ansprüche 1 bis 3, wobei das Vernetzen bei einer Temperatur zwischen 0 und 12°C durchgeführt wird.

5. Verfahren nach einem der Ansprüche 1 bis 4, wobei die vernetzte Hyaluronsäure in der Form einer viskosen wäßrigen Lösung hergestellt wird.

6. Verfahren nach Anspruch 5, wobei die Lösung eine Viskosität von 15.000 bis 40.000 cP aufweist.

7. Viskose wäßrige Lösung von vemetzter Hyaluronsäure oder deren Salz, welche für die viskose Ergänzung von Gelenken geeignet ist, wobei die vernetzte Hyaluronsäure ein Molekulargewicht von zwischen 0,7 und 8 MDa aufweist und die Lösung eine Viskosität zwischen 15.000 und 40.000 cP aufweist.

8. Viskose Lösung nach Anspruch 7, welche eine Viskosität zwischen 18.000 und 32.000 cP aufweist.

9. Verwendung einer viskosen Lösung nach Anspruch 7 oder 8 oder erhalten nach einem der Ansprüche 1 bis 6 zur Verwendung in der Behandlung von Mensch oder Tier oder in der Chirurgie.

## Revendications

1. Procédé pour préparer un acide hyaluronique (HA) réticulé ou un sel de celui-ci, en réticulant une solution aqueuse de HA par de la divinyl sulfone (DVS), ***caractérisé* en ce que** la réticulation par la DVS est réalisée à un pH compris entre 8 et 11 avec un rapport molaire DVS/HA compris entre 1 et 10% (une mole de HA étant équivalente à une unité d'anhydromonosaccharide moyenne).

2. Procédé selon la revendication 1, dans lequel un rapport de masse molaire DVS/HA compris entre 2 et 5% est utilisé.

3. Procédé selon la revendication 1 ou 2, dans lequel le HA réticulé possède un poids moléculaire compris entre 0,7 et 8 MDa.

4. Procédé selon l'une des revendications 1 à 3, dans lequel la réticulation est réalisée à une température comprise entre 0 et 12°C.

5. Procédé selon l'une des revendications 1 à 4, dans lequel l'acide hyaluronique réticulé est produit sous forme de solution aqueuse visqueuse.

6. Procédé selon la revendication 5, dans lequel la solution possède une viscosité de 15 000 à 40 000 cP.

7. Solution visqueuse d'acide hyaluronique réticulé ou d'un sel de celui-ci, convenant pour la viscosupplémentation des articulations, ledit acide hyaluronique réticulé ayant un poids moléculaire compris entre 0,7 et 8 MDa et ladite solution ayant une viscosité comprise entre 15 000 et 40 000 cP.

8. Solution visqueuse selon la revendication 7, ayant une viscosité comprise entre 18 000 et 32 000 cP.

9. Utilisation d'une solution visqueuse selon la revendication 7 ou 8, ou obtenue selon l'une des revendications 1 à 6, pour une utilisation en thérapie ou en chirurgie humaine ou vétérinaire.
